# EUROPEAN PATENT APPLICATION

(11) **EP 3 366 291 A1**
(43) Date of publication of application: **29.08.2018**
(21) Application number: 18157656.2
(22) Date of filing: 20.02.2018
(51) Int. Cl.: A61K 31/381, A61K 31/51, A61K 31/4415, A61K 31/714, A61K 47/02, A61K 47/22, A61K 47/24, A61K 47/36, A61K 47/38, A61K 9/00, A61K 9/20

(54) **SOLID ORAL PHARMACEUTICAL COMPOSITIONS COMPRISING ALPHA LIPOIC ACID AND WATER-SOLUBLE VITAMINS**

(30) Priority: 22.02.2017 TR 201702631
(71) Applicant: MONTERO GIDA SANAYI VE TICARET A.S., Istinye, Istanbul 34460 (TR)
(72) Inventor: TÜRKYILMAZ, Ali, 34460 Istanbul (TR); PEHLIVAN AKALIN, Nur, 34460 Istanbul (TR); KARACA, Gizem, 34460 Istanbul (TR)
(74) Representative: Sevinç, Erkan

(57) **Abstract**

The present invention relates to multilayer solid oral pharmaceutical compositions comprising alpha lipoic acid or a pharmaceutically acceptable salt thereof in combination with at least one water-soluble vitamin or pharmaceutically acceptable salts thereof.

## Description

### Field of Invention

The present invention relates to multilayer solid oral pharmaceutical compositions comprising alpha lipoic acid or a pharmaceutically acceptable salt thereof in combination with at least one water-soluble vitamin or pharmaceutically acceptable salts thereof.

### Background of Invention

Alpha lipoic acid (ALA) is an organosulfur compound derived from octanoic acid. ALA is made in animals normally, and is essential for aerobic metabolism. It is also manufactured and is available as a dietary supplement in some countries where it is marketed as an antioxidant, and is available as a pharmaceutical drug in other countries.

It is able to pass the blood-brain barrier and is putatively used for detoxification of mercury attached to the brain cells. It can mobilise bound mercury into the blood stream as it is a mercaptan (sulfur compound which readily binds to the mercury). ALA and perhaps vitamin B12 could make it possible for other chelators to remove mercury safely out of the body and could perhaps one day be used as a treatment for autism. Because ALA is related to cellular uptake of glucose and it is both soluble in water and fat, it is being used for treatment in diabetes. It may be helpful for people with Alzheimer's disease or Parkinson's disease.

Orally-administered tablets of alpha lipoic acid (thioctic acid) are commercially available under the name Thioctacid®. Its chemical name is (R)-5-(1,2-Dithiolan-3-yl)pentanoic acid and it has the chemical structure shown in Formula I.

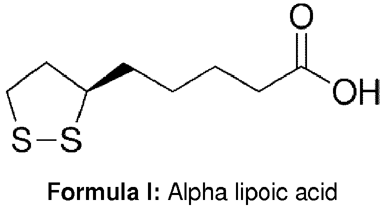

On the other hand, vitamins are classified as either fat soluble (vitamins A, D, E and K) or water soluble (vitamins B and C). This difference between the two groups is very important since it determines how each vitamin acts within the body.

B vitamins are a class of water-soluble vitamins that play important roles in cell metabolism. Though these vitamins share similar names, research shows that they are chemically distinct vitamins that often coexist in the same foods.

Thiamine, also known as vitamin B1, is a vitamin found in food and used as a dietary supplement. As a supplement it is used to treat and prevent thiamine deficiency and disorders that result from it including beriberi and Korsakoff's syndrome. Thiamine plays a key role in intracellular glucose metabolism and it is thought that thiamine inhibits the effect of glucose and insulin on arterial smooth muscle cell proliferation. Thiamine plays an important role in helping the body convert carbohydrates and fat into energy. It is essential for normal growth and development and helps to maintain proper functioning of the heart and the nervous and digestive systems. Thiamine cannot be stored in the body; however, once absorbed, the vitamin is concentrated in muscle tissue.

Its chemical name is 3-[(4-amino-2-methylpyrimidin-5-yl)methyl]-5-(2-hydroxyethyl)-4-methyl-1,3-thiazol-3-ium and it has the chemical structure shown in Formula II.

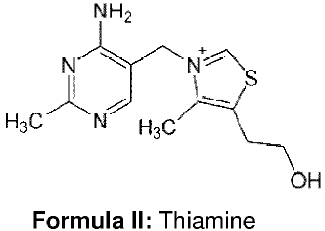

Pyridoxine is the 4-methanol form of vitamin B6 found commonly in food and used as dietary supplement. As a supplement it is used to treat and prevent pyridoxine deficiency, sideroblastic anaemia, pyridoxine-dependent epilepsy, certain metabolic disorders, problems from isoniazid, and mushroom poisoning. It is converted to pyridoxal 5-phosphate in the body. Pyridoxal 5-phosphate is a coenzyme for synthesis of amino acids, neurotransmitters (serotonin, norepinephrine), sphingolipids, aminolevulinic acid.

Its chemical name is 4,5-bis(hydroxymethyl)-2-methylpyridin-3-ol and it has the chemical structure shown in Formula III.

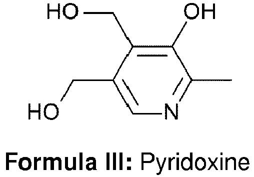

Cyanocobalamin, commonly known as Vitamin B12, is the most chemically complex of all the vitamins. Cyanocobalamin cannot be made by plants or by animals, as the only type of organisms that have the enzymes required for the synthesis of cyanocobalamin are bacteria and archaea. Higher plants do not concentrate cyanocobalamin from the soil and so are a poor source of the substance as compared with animal tissues. Cyanocobalamin is naturally found in foods including meat (especially liver and shellfish), eggs, and milk products. It is usually prescribed after surgical removal of part or all of the stomach or intestine to ensure adequate serum levels of vitamin B12. It is also used to treat pernicious anemia, vitamin B12 deficiency (due to low intake from food), thyrotoxicosis, hemorrhage, malignancy, liver disease and kidney disease.

Its chemical name is [(1R,2R,3S,4S,8S,9S,14S,18R,19R)-4,9,14-tris(2-carbamoylethyl)-3,8,19-tris(carbamoylmethyl)-18-(2-{[(2R)-2-[({[(2R,3S,4R,5S)-5-(5,6-dimethyl-1H-1,3-benzodiazol-1-yl)-4-hydroxy-2-(hydroxymethyl)oxolan-3-yl]oxy}(hydroxy)phosphoryl)oxy] propyl]carbamoyl}ethyl)-2,3,6,8,13,13,16,18-octamethyl-20,21,22,23-tetraazapentacyclo [15.2.1.1²,⁵.1⁷,¹⁰.1¹²,¹⁵]tricosa-5(23),6,10(22),11,15(21),16-hexaen-20-yl]cobaltcarbonitrile and it has the chemical structure shown in Formula IV.

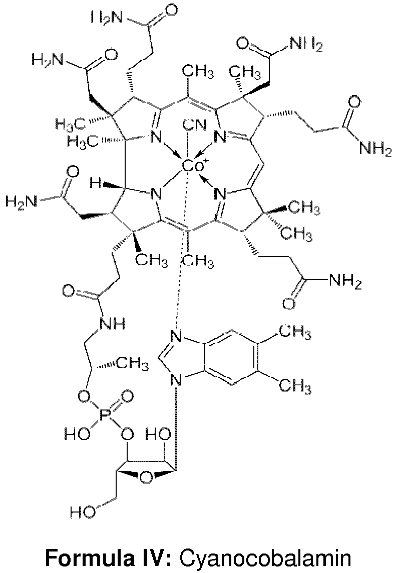

It is a well-known technic to combine vitamins, vitamin-like compounds and minerals in the same dosage form in order to enhance the bioavailability for the same therapeutic effect or in order to come up with a multi-indication dosage form. Combination therapy also reduces the daily dosages to be taken by patients and simplifies dosing schedule thereby increases patient compliance. Prior art has various examples presenting pharmaceutical formulations or treatment methods in which lipoic acid and vitamins are combined.

One of the main problems of lipoic acid formulations is the low solubility (125 mg/L in water) of this active agent and accordingly insufficient dissolution of the related composition. This problem is also prevalent in combination formulations comprising lipoic acid. In the state of art, the presence of at least one disintegrant in the formulation is seen inevitable to solve this problem.

Patent application numbered US2002151578A1, which is one of these examples, mentions a formulation based on lipoic acid and its use for oral administrations. It is also stated that the formulation can be a multilayer dosage form comprising a combination of lipoic acid and various vitamins and that it comprises tablet excipients such as binders, fillers, glidants, lubricants, disintegrants and coatings resistant to gastric juice. In the application, talc is one of the suggested glidants or lubricants to be used in the core tablet formulation.

Especially for sustained-release or modified-release formulations of lipoic acid, the use of a dissolution retardant is the first thing coming to mind; however, it reduces the dissolution and disintegration of lipoic acid which has already low solubility. Therefore, additional supplementary excipients are supposed to be used or already existing excipient are overused in such cases to enhance the dissolution and disintegration profiles.

Patent applications numbered CN104546782A and CN1868463A are facing the same problem mentioned above.

In the application numbered CN104546782A, enteric-coated pellets of a lipoic acid formulation are subjected to the invention. The formulation comprises microcrystalline cellulose as disintegrant to provide the required dissolution profile. Besides, it is stated that the amount of the enteric coating is between 20-40% in the composition.

On the other side, the application numbered CN1868463A mentions a bilayer sustained-release tablet of a lipoic acid formulation. To provide the sustained-release of the active agent, a dissolution retarder, which is preferably talc, is used. Two disintegrants, which are sodium carboxymethyl starch and microcrystalline cellulose, are included in the composition to tolerate the disintegration profile.

Thus, there is no teaching, suggestion or motivation in the prior art about how to develop a combination dosage form of lipoic acid and water-soluble vitamins ensuring the required dissolution and disintegration profiles without unnecessary use or overuse of excipients. Therefore, there is still a need for more simple and effective formulations of these active agents which eliminate this complexity and which enhance reproducibility, patient compliance and bioavailability of the final pharmaceutical product.

### Objects and Brief Description of the Invention

The main object of the present invention is to obtain combination formulations, comprising alpha lipoic acid or a pharmaceutically acceptable salt thereof in combination with at least one water soluble vitamin or pharmaceutically acceptable salt thereof, eliminating all aforesaid problems and bringing additional advantages to the relevant prior art.

Another object of the present invention is to obtain combination formulations of alpha lipoic acid and water-soluble vitamins with patient compliance.

Another object of the present invention is to obtain combination formulations of alpha lipoic acid and water-soluble vitamins with high stability and bioavailability.

A further object of the present invention is to develop combination formulations of alpha lipoic acid and water-soluble vitamins having improved level of dissolution and disintegration rates.

Another object of the present invention is to provide a multilayer dosage form which comprises alpha lipoic acid and water-soluble vitamins in separate layers.

Another object of the present invention is to provide a multilayer dosage form in which alpha lipoic acid layer is free of talc and disintegrant.

Yet, another object of the present invention is to provide a bilayer tablet of alpha lipoic acid and water-soluble vitamins comprising significantly reduced amount of enteric coating.

### Detailed Description of Invention

In accordance with the objects outlined above, detailed features of the present invention are given herein.

List of figures:
**Figure 1** shows the lateral cross-sectional area of an embodiment of the oral pharmaceutical composition subjected to the invention.
**Figure 2** shows the lateral cross-sectional area of another embodiment of the oral pharmaceutical composition subjected to the invention.
**Figure 3** shows the lateral cross-sectional area of an embodiment of the oral pharmaceutical composition subjected to the invention.

The present invention relates to an oral pharmaceutical composition comprising alpha lipoic acid or a pharmaceutically acceptable salt thereof in combination with at least one water-soluble vitamin or pharmaceutically acceptable salt thereof as active agents.

According to this preferred embodiment, the composition comprises
- a first layer (1) comprising alpha lipoic acid or pharmaceutically acceptable salt thereof and
- a second layer (2) comprising water-soluble vitamins or pharmaceutically acceptable salts thereof.

According to this preferred embodiment, the composition further comprises an enteric coating (3).

According to the preferred embodiment, the first layer (1) is free of disintegrant. According to this preferred embodiment, the first layer (1) is also free of talc to tolerate the lack of disintegrant and to balance dissolution and disintegration profiles of the layer since it is known that talc is widely used as a dissolution retardant. Besides, talc of high amount is known to increase risk of ovarian cancer in women (Handbook of Pharmaceutical Excipients, sixth edition, page 730). It has been seen that stability and bioavailability of the final product is still maintained, even enhanced, considering the following embodiments, despite the absence of disintegrant and talc.

According to the preferred embodiment, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, gastric disintegrating tablet, pill, capsule, oral granule, powder, coated bead system, microsphere, tablet in tablet, inlay tablet, dragee, sachet or orally administrable film.

The composition is preferably in the form of bilayer tablet.

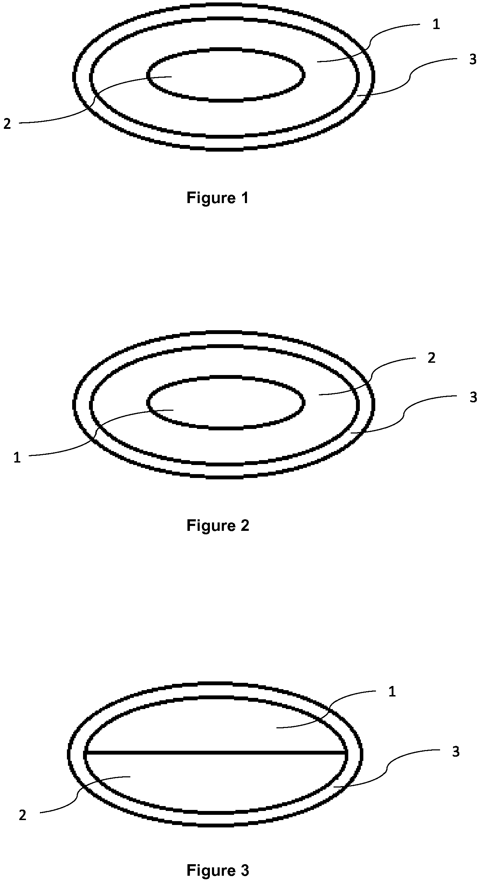

According to one embodiment, the first layer (1) covers completely or partially the second layer (2). According to this embodiment, the enteric coating (3) covers the first layer (1) as seen in Figure 1.

According to another embodiment, the second layer (2) covers completely or partially the first layer (1). According to this embodiment, the enteric coating (3) covers the second layer (2) as seen in Figure 2.

According to an another embodiment, the first layer (1) and the second layer (2) are adjacent collaterally. According to this embodiment, the enteric coating (3) covers the first layer (1) and the second layer (2) as seen in Figure 3.

According to the preferred embodiment, particle size distributions of these active agents play a significant role for the qualification of the composition subjected to the invention. As used herein, 'particle size distribution' means the cumulative volume size distribution as tested by any conventionally accepted method such as the laser diffraction method (Malvern analysis).

Laser diffraction measures particle size distributions by measuring the angular variation in intensity of light scattered as a laser beam passes through a dispersed particulate sample. Large particles scatter light at small angles relative to the laser beam and small particles scatter light at large angles. The angular scattering intensity data is then analyzed to calculate the size of the particles responsible for creating the scattering. The particle size is reported as a volume equivalent sphere diameter.

According to this measuring method, the D50 value is the size in microns that splits the distribution with half above and half below this diameter. Similarly, 90% of the distribution lies below the D90 value, and 10% of the distribution lies below the D10 value.

In the preferred embodiment of the invention, the D50 value of the particles of water soluble vitamins or pharmaceutically acceptable salts thereof is lower than 500 µm, more preferably this value is ranging between 1 and 200 µm.

According to this preferred embodiment, the D90 value of the particles of water soluble vitamins or pharmaceutically acceptable salts thereof is lower than 700 µm, more preferably this value is ranging between 1 and 300 µm.

According to these preferred embodiments, the D10 value of the particles of water soluble vitamins or pharmaceutically acceptable salts thereof is lower than 100 µm, more preferably this value is ranging between 0.1 and 20 µm.

According to the preferred embodiment, the said water-soluble vitamins comprise B vitamins.

According to this preferred embodiment of the invention, the composition comprises alpha lipoic acid in combination with B vitamins or pharmaceutically acceptable salts thereof. According the most preferred embodiment, the said B vitamins or pharmaceutically acceptable salts thereof are selected from thiamine hydrochloride (vitamin B1), pyridoxine hydrochloride (vitamin B6), cyanocobalamin (vitamin B12) or mixtures thereof.

According to the preferred embodiment of the invention, the composition comprises alpha lipoic acid in the amount of 1-60% by weight of the total composition. Preferably this amount is between 5-50% by weight of the total composition. More preferably alpha lipoic acid is present between 20-30% by weight in the total composition.

According to the preferred embodiment, the amount of thiamine hydrochloride is between 1-50% by weight of the total composition. Preferably this amount is between 5-40% by weight of the total composition. More preferably thiamine hydrochloride is present between 15-25% by weight in the total composition.

According to the preferred embodiment, the amount of pyridoxine hydrochloride is between 1-50% by weight of the total composition. Preferably this amount is between 5-40% by weight of the total composition. More preferably pyridoxine hydrochloride is present between 15-25% by weight in the total composition.

According to the preferred embodiment, the amount of cyanocobalamin is between 0.01-1% by weight of the total composition. Preferably this amount is between 0.05-0.5% by weight of the total composition.

In one embodiment of the invention, the weight of the composition is between 500 and 1500 mg, preferably it is 1150 mg.

Alpha lipoic acid is present in an amount of 100 to 1000 mg, preferably 200 to 500 mg and more preferably 300 mg in the composition.

Thiamine hydrochloride is present in an amount of 100 to 1000 mg, preferably 150 to 500 mg and more preferably 250 mg in the composition.

Pyridoxine hydrochloride is present in an amount of 100 to 1000 mg, preferably 150 to 500 mg and more preferably 250 mg in the composition.

Cyanocobalamin is present in an amount of 0.1 to 10 mg, preferably 0.5 to 5 mg and more preferably 1 mg in the composition.

According to any of these embodiments, the first layer (1) comprises alpha lipoic acid and at least one excipient selected from diluents, binders, lubricants, glidants or mixtures thereof.

The amount of alpha lipoic acid is between 20-95%, preferably 40-90%, more preferably 70-80% by weight of the first layer (1).

According to one embodiment of the invention, the first layer (1) of the composition comprises at least one diluent which is selected from the group comprising lactose monohydrate, lactose, microcrystalline cellulose, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment of the invention, the first layer (1) comprises microcrystalline cellulose as diluent. The amount of microcrystalline cellulose is between 5-50%, preferably 5-40%, more preferably 10-30% by weight of the first layer (1).

According to one embodiment of the invention, the first layer (1) of the composition comprises at least one binder which is selected from the group comprising polyvinylpyrrolidone (PVP, povidone), microcrystalline cellulose, copovidone, copolyvidone, polyvinylpyrrolidone K30 (povidone K30), carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to the preferred embodiment of the invention, the first layer (1) comprises hydroxypropyl cellulose as binder. The amount of hydroxypropyl cellulose is between 0.1-10%, preferably 0.5-3% by weight of the first layer (1).

According to one embodiment of the invention, the first layer (1) of the composition comprises at least one lubricant and at least one glidant which are selected from the group comprising sodium lauryl sulfate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulfate, fumaric acid, zinc stearate, stearic acid, hydrogenated natural oils, silica, paraffin or mixtures thereof.

According to the preferred embodiment of the invention, the first layer (1) comprises magnesium stearate as lubricant and colloidal silicon dioxide as glidant. The amount of magnesium stearate is between 1-10%, preferably 2-6% by weight of the first layer (1). The amount of colloidal silicon dioxide is between 0.05-3%, preferably 0.1-1% by weight of the first layer (1).

According to any of these embodiments, the second layer (2) comprises thiamine hydrochloride (vitamin B1), pyridoxine hydrochloride (vitamin B6), cyanocobalamin (vitamin B12) and at least one excipient selected from disintegrants, binders, lubricants or mixtures thereof.

The amount of thiamine hydrochloride is between 10-60%, preferably 20-50%, more preferably 30-40% by weight of the second layer (2).

The amount of pyridoxine hydrochloride is between 10-60%, preferably 20-50%, more preferably 30-40% by weight of the second layer (2).

The amount of cyanocobalamin is between 0.01-2%, preferably 0.05-1%, more preferably 0.1-0.2% by weight of the second layer (2).

According to the preferred embodiment, the second layer (2) is free of diluent.

It is a known fact that water solubility of cyanocobalamin is much lower than the water solubilities of thiamine hydrochloride and pyridoxine hydrochloride. This difference makes difficult to provide all the B vitamins in one single layer and to achieve required dissolution profiles of these vitamins at the same time. Within the scope of the present invention; the second layer (2) is formulated to eliminate this problem and thus to enhance the bioavailability.

In the preferred embodiment of the invention, the D50 value of the thiamine hydrochloride particles is lower than 100 µm, more preferably this value is ranging between 1 and 10 µm.

In the preferred embodiment of the invention, the D50 value of the pyridoxine hydrochloride particles is lower than 500 µm, more preferably this value is ranging between 50 and 200 µm.

In the preferred embodiment of the invention, the D50 value of the cyanocobalamin particles is lower than 300 µm, more preferably this value is ranging between 10 and 100 µm.

It has been seen that the content uniformity of the second layer (2) is perfectly ensured between these preferable ranges of B vitamins. Besides, relative standard deviations of the particle sizes of these vitamins are reduced according to these preferable ranges.

Accordingly, in the preferred embodiment of the invention, the ratio of the D50 value of the thiamine hydrochloride particles to the D50 value of cyanocobalamin particles is in the range of 1:0.5 and 1:20 and preferably 1:2 and 1:8.

According to this preferred embodiment of the invention, the ratio of the D50 value of the pyridoxine hydrochloride particles to the D50 value of cyanocobalamin particles is in the range of 20:1 and 0.5:1 and preferably 5:1 and 1:1.

It has been seen that these selections of D50 value ranges and ratio ranges of B vitamins surprisingly enhance tablet compressibility and accordingly patient compliance. Besides, they also assure stability during the shelf life, homogenous and efficient dissolution and the bioavailability.

According to one embodiment of the invention, the second layer (2) of the composition comprises at least one disintegrant which is selected from the group comprising croscarmellose sodium, sodium starch glycolate, microcrystalline cellulose, sodium carbonate, hydroxylpropyl cellulose (HPC), cross-linked polyvinylpyrrolidone (crospovidone), copovidon, polycarbophil, low-substitue poloxamer, alginic acid and alginates, ion-exchange resins, magnesium aluminum silica, sodium carboxy methyl cellulose, carboxy methyl cellulose calcium, docusate sodium, guar gum, polyacrylin potasium, sodium alginate, sodium glycine carbonate or mixtures thereof.

According to the preferred embodiment of the invention, the second layer (2) comprises two disintegrants which are sodium starch glycolate and microcrystalline cellulose. The amount of sodium starch glycolate is between 1-30%, preferably 5-20% by weight of the second layer (2). The amount of microcrystalline cellulose is between 1-30%, preferably 5-20% by weight of the second layer (2).

It has been seen that particle sizes of sodium starch glycolate and microcrystalline cellulose surprisingly play a significant role in the dissolution profile and accordingly in the bioavailability.

According to the preferred embodiment of the invention, the D50 value of the sodium starch glycolate particles is lower than 200 µm, more preferably this value is ranging between 10 and 100 µm.

According to the preferred embodiment of the invention, the D50 value of the microcrystalline cellulose particles is lower than 400 µm, more preferably this value is ranging between 50 and 200 µm.

Accordingly, in the preferred embodiment of the invention, the ratio of the D50 value of the sodium starch glycolate particles to the D50 value of microcrystalline cellulose particles is in the range of 1:0.5 and 1:6 and preferably 1:1.5 and 1:4.

According to one embodiment of the invention, the second layer (2) of the composition comprises at least one binder which is selected from the group comprising polyvinylpyrrolidone (PVP, povidone), microcrystalline cellulose, copovidone, copolyvidone, polyvinylpyrrolidone K30 (povidone K30), carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to the preferred embodiment of the invention, the second layer (2) comprises hydroxypropyl cellulose as binder. The amount of hydroxypropyl cellulose is between 1-20%, preferably 5-10% by weight of the second layer (2).

According to one embodiment of the invention, the second layer (2) of the composition comprises at least one lubricant which is selected from the group comprising sodium lauryl sulfate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulfate, fumaric acid, zinc stearate, stearic acid, hydrogenated natural oils, silica, paraffin or mixtures thereof.

According to the preferred embodiment of the invention, the second layer (2) comprises magnesium stearate as lubricant. The amount of magnesium stearate is between 0.5-10%, preferably 1-5% by weight of the second layer (2).

According to the preferred embodiment of the invention, the composition further comprises at least one enteric coating (3) to prevent the tablet dissolution or disintegration in the gastric environment. Suitable enteric coating (3) ingredients are selected from the group comprising hypromellose, lactose monohydrate, hydroxypropyl cellulose, polyvinyl alcohol (PVA), ethyl cellulose, polyethylene glycol (PEG), talc, triethyl citrate, polyvinyl alcohol-polyethylene glycol copolymers (Kollicoat® IR), ethyl cellulose dispersions (Surelease®), polyvinylprolidone, polyvinylprolidone-vinyl acetate copolymer (PVP-VA), all kinds of Opadry®, pigments, dyes, titanium dioxide, iron oxide or mixtures thereof.

According to one embodiment, the enteric coating (3) comprises ethyl cellulose, talc, titanium dioxide, triethyl citrate, iron oxide, blue pigment or mixtures thereof.

Preferably, the enteric coating (3) is ethyl cellulose based Sheffcoat™. The amount of the enteric coating (3) is 1-10%, preferably %3-6 by weight of the total composition.

Following the conducted analyses, it has been seen that the enteric coating (3) maintains its stability on the core tablet and does not dissolve in the gastric environment even in this distinctly low ratio range. Accordingly, the reduced amount of the enteric coating (3) covering the core tablet shall increase patient compliance due to the reduced size of the final pharmaceutical product.

According to these embodiments, the composition comprises;
- 20-95% of alpha lipoic acid,
- 5-50% of microcrystalline cellulose,
- 0.1-10% of hydroxypropyl cellulose,
- 0.05-3% of colloidal silicon dioxide,
- 1-10% of magnesium stearate by weight of the first layer (1);
- 10-60% of thiamine hydrochloride (vitamin B1),
- 10-60% of pyridoxine hydrochloride (vitamin B6),
- 0.01-2% of cyanocobalamin (vitamin B12),
- 1-20% of hydroxypropyl cellulose,
- 1-30% of sodium starch glycolate,
- 1-30% of microcrystalline cellulose,
- 0.5-10% of magnesium stearate by weight of the second layer (2) and
- 1-10% enteric coating (3) by weight of the total composition.

According to one embodiment, the composition comprises;
- 10-40% of alpha lipoic acid,
- 5-30% of microcrystalline cellulose,
- 1-10% of hydroxypropyl cellulose,
- 0.1-1 % of colloidal silicon dioxide,
- 1-5% of magnesium stearate
- 10-30% of thiamine hydrochloride (vitamin B1),
- 10-30% of pyridoxine hydrochloride (vitamin B6),
- 0.01-1% of cyanocobalamin (vitamin B12),
- 1-20% of sodium starch glycolate,
- 1-10% enteric coating (3) by weight of the total composition.

These analytically selected ratios ensure the required effective doses for the treatment and enhance the stability and dissolution profile of the enteric film coated bilayer tablet subjected to the invention.

According to all these embodiments, the below given formulations can be used in the solid oral pharmaceutical composition subjected to the invention. These examples are not limiting the scope of the present invention and should be considered under the light of the foregoing detailed disclosure.

### Example 1: Ratios by weight of the total composition (coated bilayer tablet)

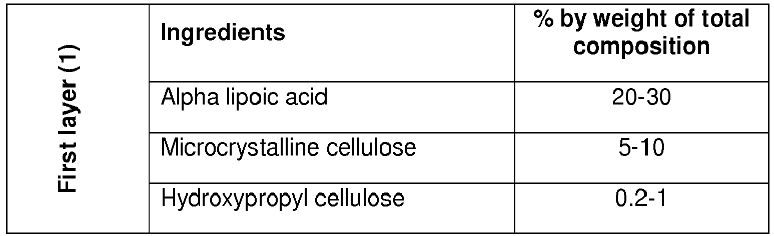

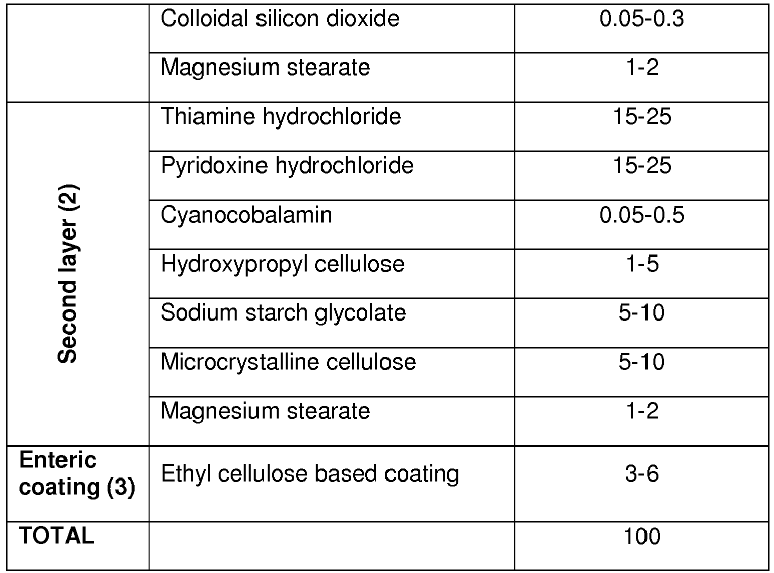

### Example 2: Ratios by weight of the total composition (coated bilayer tablet)

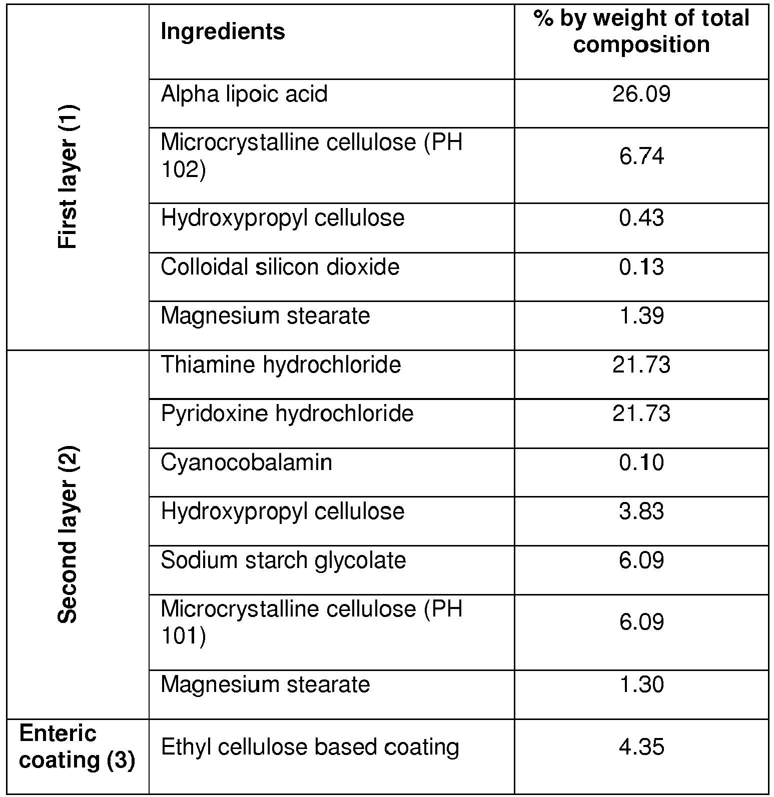

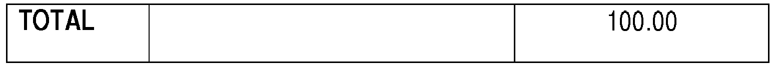

The pharmaceutical compositions mentioned above are prepared by following these steps:
- mixing thiamine hydrochloride, pyridoxine hydrochloride, cyanocobalamin, microcrystalline cellulose reserved for the second layer (2) (PH 101), sodium starch glycolate and three-fourths of hydroxypropyl cellulose reserved for the second layer (2) together to prepare a powder mixture comprising B vitamins
- mixing one fourth of hydroxypropyl cellulose reserved for the second layer (2) and ethyl alcohol to prepare a granulation solution
- granulating the powder mixture comprising B vitamins with the granulation solution (wet granulation)
- sieving the granules comprising B vitamins through an 8mm mesh and drying them
- sieving the dried granules again through a 1.5mm mesh
- adding magnesium stearate reserved for the second layer (2) to the sieved granules and mixing this total mixture which will form the second layer (2)
- on the other side, sieving alpha lipoic acid, hydroxypropyl cellulose reserved for the first layer (1), colloidal silicon dioxide and microcrystalline cellulose reserved for the first layer (1) (PH 102) through a 630µm mesh
- mixing these together to prepare a powder mixture comprising alpha lipoic acid
- adding magnesium stearate reserved for the first layer (1) to the powder mixture comprising alpha lipoic acid and mixing the total mixture which will form the first layer (1)
- compressing these two total mixtures together into bilayer tablets
- preparing an enteric coating (3) solution and coating the tablets with this solution

## Claims

1. A solid oral pharmaceutical composition comprising a first layer (1) containing alpha lipoic acid or a pharmaceutically acceptable salt thereof and a second layer (2) containing at least one water-soluble vitamin or a pharmaceutically acceptable salt thereof; wherein the first layer (1) is free of disintegrant.

2. The solid oral pharmaceutical composition according to claim 1, wherein the first layer (1) is also free of talc.

3. The solid oral pharmaceutical composition according to claim 1 or 2, wherein the composition in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, gastric disintegrating tablet, pill, capsule, oral granule, powder, coated bead system, microsphere, tablet in tablet, inlay tablet, dragee, sachet or orally administrable film.

4. The solid oral pharmaceutical composition according to claim 3, wherein the composition is in the form of bilayer tablet.

5. The solid oral pharmaceutical composition according to any one of the preceding claims, wherein the first layer (1) covers completely or partially the second layer (2) or the second layer (2) covers completely or partially the first layer (1) or the first layer (1) and the second layer (2) are adjacent collaterally.

6. The solid oral pharmaceutical composition according to any one of the preceding claims, wherein the said water-soluble vitamins comprise B vitamins.

7. The solid oral pharmaceutical composition according to claim 6, wherein the said B vitamins or pharmaceutically acceptable salts thereof are selected from thiamine hydrochloride, pyridoxine hydrochloride, cyanocobalamin or mixtures thereof.

8. The solid oral pharmaceutical composition according to claim 7, wherein the D50 value of the thiamine hydrochloride particles is lower than 100 µm, preferably it is ranging between 1 and 10 µm.

9. The solid oral pharmaceutical composition according to claim 7, the D50 value of the pyridoxine hydrochloride particles is lower than 500 µm, preferably it is ranging between 50 and 200 µm.

10. The solid oral pharmaceutical composition according to claim 7, the D50 value of the cyanocobalamin particles is lower than 300 µm, preferably it is ranging between 10 and 100 µm.

11. The solid oral pharmaceutical composition according to claim 7, wherein the ratio of the D50 value of the thiamine hydrochloride particles to the D50 value of cyanocobalamin particles is in the range of 1:0.5 and 1:20, preferably it is in the range of 1:2 and 1:8.

12. The solid oral pharmaceutical composition according to claim 7, wherein the ratio of the D50 value of the pyridoxine hydrochloride particles to the D50 value of cyanocobalamin particles is in the range of 20:1 and 0.5:1, preferably it is in the range of 5:1 and 1:1.

13. The solid oral pharmaceutical composition according to any one of the preceding claims, wherein the composition further comprises an enteric coating (3).

14. The solid oral pharmaceutical composition according to any one of the preceding claims, wherein the first layer (1) further comprises at least one excipient selected from diluents, binders, lubricants, glidants or mixtures thereof.

15. The solid oral pharmaceutical composition according to any one of the preceding claims, wherein the second layer (2) further comprises at least one excipient selected from disintegrants, binders, lubricants or mixtures thereof.

16. The oral pharmaceutical composition according to any one of the preceding claims, wherein the composition comprises;
- 20-95% of alpha lipoic acid,
- 5-50% of microcrystalline cellulose,
- 0.1-10% of hydroxypropyl cellulose,
- 0.05-3% of colloidal silicon dioxide,
- 1-10% of magnesium stearate by weight of the first layer (1);
- 10-60% of thiamine hydrochloride,
- 10-60% of pyridoxine hydrochloride,
- 0.01-2% of cyanocobalamin,
- 1-20% of hydroxypropyl cellulose,
- 1-30% of sodium starch glycolate,
- 1-30% of microcrystalline cellulose,
- 0.5-10% of magnesium stearate by weight of the second layer (2) and
- 1-10% enteric coating (3) by weight of the total composition.

17. A process for preparing the oral pharmaceutical composition according to claim 16, comprising the following steps:
- mixing thiamine hydrochloride, pyridoxine hydrochloride, cyanocobalamin, microcrystalline cellulose reserved for the second layer (2), sodium starch glycolate and three-fourths of hydroxypropyl cellulose reserved for the second layer (2) together to prepare a powder mixture comprising B vitamins
- mixing one fourth of hydroxypropyl cellulose reserved for the second layer (2) and ethyl alcohol to prepare a granulation solution
- granulating the powder mixture comprising B vitamins with the granulation solution (wet granulation)
- sieving the granules comprising B vitamins and drying them
- sieving the dried granules again
- adding magnesium stearate reserved for the second layer (2) to the sieved granules and mixing this total mixture which will form the second layer (2)
- on the other side, sieving alpha lipoic acid, hydroxypropyl cellulose reserved for the first layer (1), colloidal silicon dioxide and microcrystalline cellulose reserved for the first layer (1)
- mixing these together to prepare a powder mixture comprising alpha lipoic acid
- adding magnesium stearate reserved for the first layer (1) to the powder mixture comprising alpha lipoic acid and mixing the total mixture which will form the first layer (1)
- compressing these two total mixtures together into bilayer tablets
- preparing an enteric coating (3) solution and coating the tablets with this solution
